# EUROPEAN PATENT SPECIFICATION

(11) **EP 4 327 741 B1**
(45) Date of publication and mention of the grant of the patent: **13.05.2026**
(21) Application number: 22192512.6
(22) Date of filing: 27.08.2022
(51) Int. Cl.: A61B 5/145, A61B 5/00

(54) **PROCESS FOR IN SITU TESTING AN ANALYTE MONITOR, AND ANALYTE MONITOR**
VERFAHREN ZUR IN-SITU PRÜFUNG EINES ANALYTMONITORS UND ANALYTMONITOR
PROCÉDÉ DE TEST IN SITU D'UN MONITEUR D'ANALYTE ET MONITEUR D'ANALYTE

(43) Date of publication of application: 28.02.2024
(73) Proprietor: Eclypia, 38000 Grenoble (FR)
(72) Inventor: MONPEURT, Cyrielle, 38000 GRENOBLE (FR); BLANC, Romain, 38000 GRENOBLE (FR)
(74) Representative: Fidal Innovation

(56) References cited:
- EP-A2- 1 205 753
- US-A1- 2017 042 428
- US-A1- 2018 055 370
- US-A1- 2022 257 154

## Description

### FIELD OF THE INVENTION

The present invention relates to an analyte monitor based on photoacoustic or photothermal detection.

More specifically, the invention relates to a process for testing in situ such an analyte monitor.

### TECHNOLOGICAL BACKGROUND

In the field of sensors for human beings or animals, non-invasive sensors based on photoacoustic or photothermal detection are known. By way of an example, glycemia can be measured with a photoacoustic or a photothermal sensor, such as that of US 2022/257,154.

A zone of interest of a medium to be analysed, called target, is irradiated by means of a laser beam of wavelength and modulation frequency chosen according to the parameter of interest to be measured. The laser beam is absorbed by the target to a depth which depends on the structuring of the target. The absorption of light energy leads to local heating of the target. In response to this heating, a thermal wave with a frequency equal to the modulation frequency of the laser is generated in the target. This wave propagates in the target and in particular to the outer surface of the target.

In the case of a photothermal sensor, the thermal wave is directly detected and analysed. Photoacoustic detection exploits the fact that the thermal wave is associated with a pressure wave of frequency identical to the modulation frequency. In the case of indirect photoacoustic detection, the pressure wave generated in the fluid external environment is detected when the thermal wave generated in the target reaches, after propagation, the interface between the target and its fluid external environment.

Photoacoustic detection has many advantages compared to other detection techniques, among which the fact that the transduction is orthogonal: the optical signal entering the medium to be analysed is converted into an acoustic signal which is very specific to the phenomenon to be observed and which makes it possible to use inexpensive and miniaturized sensors.

The difficulty of photoacoustic or photothermal detection comes (among others) from:
- the number of parameters generally influencing the detected signal and,
- for certain analytes of interest present in low concentration in the medium to be analysed, the low proportion of the detected signal specific to each of these parameters of interest.

In a stratified material, in order to be able to deduce from the photoacoustic or photothermal signal the concentration of a given layer in an analyte of interest, it is thus necessary to know all the other parameters influencing this signal. In particular, it is necessary to know the structuring of the material, i.e. the thicknesses of the different layers constituting the material, their respective physico-chemical compositions (with the exception of the parameter of interest to be measured), as well as possibly their thermal conductivities or even the thermal resistance associated with each interface between two successive layers.

Calibrating once and for all (or at least for a significant period of use) a sensor based on photoacoustic or photothermal detection is only possible if only the parameter of interest varies in the target. On the other hand, when the characteristics of the stratified medium to be analysed vary, it is necessary to carry out a calibration of the sensor regularly to obtain a measurement with acceptable accuracy.

This problem arises more particularly for sensors intended for use on living organisms. For example, in the case of a non-invasive interstitial blood glucose sensor, the calibration of a non-invasive sensor based on photoacoustic or photothermal detection can only be achieved with limited accuracy because the composition of the skin varies not only from one patient to another but also over time for a given patient.

Before even thinking of calibrating the analyte monitor, it is necessary to test its correct operation, i.e. to check that there is for example no failure of one of its components or no problem with the positioning of the sensor.

For example, in the case of a glycemia sensor comprising a cuff positioned on the arm of a patient, the cuff may not be tight on the arm or may move due to the patient movements so that its position doesn't allow measuring glycemia. The part of the signal associated with glycemia may in such cases be drowned in noise.

In such cases, even if the glycemia sensor provides a measurement, this measurement is not reliable.

As a consequence, an in situ testing process for repeatedly testing in situ an analyte monitor based on photoacoustic or photothermal detection is necessary.

In the field of photoacoustic sensors, in general, a calibration procedure is carried out with a reference sample or a calibration unit. This calibration can at the same time be exploited as a testing procedure.

US11275059 describes such an in situ calibration unit. In a particular embodiment, the calibration unit is integrated in the photoacoustic sensor. However, bearing in mind miniaturization, weight and/or management of the complexity and of the cost of the material, embedding an additional calibration unit or an additional reference in an analyte can be a major problem.

The invention aims at solving the technical problem of the in situ testing of an analyte monitor based on photoacoustic or photothermal detection with a reduced impact on the size and/or the complexity of the analyte monitor.

### SUMMARY OF THE INVENTION

The invention is defined by the appended claims. The invention relates to a process for in situ testing an analyte monitor based on photoacoustic or photothermal detection comprising:
a) providing an analyte monitor based on photoacoustic or photothermal detection at a measurement position relative to a target;
b) setting an irradiation configuration of a light source of the analyte monitor comprising at least one triplet of irradiation parameters comprising a modulation frequency, a wavelength and an optical power;
c) irradiating the target with the light source of the analyte monitor configured with the irradiation configuration at a plurality of irradiation time points and measuring for each time point at least an amplitude and a phase of an acoustic or thermal wave generated in response to the irradiation for at least one triplet of irradiation parameters of the irradiation configuration;
d) calculating with a processor of the analyte monitor at least one correlation coefficient between the amplitude and the phase for at least one triplet of irradiation parameters of the irradiation configuration over a window period comprising at least two irradiation time points of said plurality of irradiation time points;
e) comparing with the processor of the analyte monitor at least one of the at least one correlation coefficient with at least one threshold value;
f) providing with a processor of the analyte monitor a test result based on the result of the comparison.

The inventors have indeed observed that when a significant signal is to be observed (for example a signal other than noise), the amplitude and the phase of the photoacoustic or photothermal signal are generally correlated (positively or negatively). If a correlation coefficient, or the absolute value of this correlation coefficient, between the amplitude and the phase of the signal for a given triplet of irradiation parameters is higher than a threshold value, this is an indication of the good functioning of the analyte monitor. On the contrary, if a correlation coefficient, or the absolute value of this correlation coefficient, between the amplitude and the phase of the signal for a given triplet of irradiation parameters is lower than a threshold value, we can suspect that the analyte monitor is not correctly operated. Hence, the process according to the invention allows to test in situ the correct operation of the analyte monitor without requiring any additional component or a reference.

In a particular embodiment of the process for in situ testing an analyte monitor based on photoacoustic or photothermal detection, if the absolute value of at least one correlation coefficient is higher than a threshold value, the test result is an authorisation to proceed with at least one subsequent measurement with the analyte monitor.

In this case, an in situ test can be repeatedly carried out, for example periodically, and each time the comparison leads to the conclusion that the analyte monitor is working properly, the analyte monitor is made available for measurements until the next in situ test. The control of the analyte monitor proper functioning can thus be automated and carried out continuously in situ without a patient wearing the analyte monitor having to worry about it or an operator operating the analyte monitor in a complex environment to have to interfere with this environment. A patient or an operator can as a consequence rely on the analyte monitor while the measurements are authorized.

In a particular embodiment of the process for in situ testing an analyte monitor based on photoacoustic or photothermal detection, if the absolute value of at least one correlation coefficient is lower than a threshold value, the test result comprises displaying an alert chosen in the list : message on a user interface of the analyte monitor or by emailing to check the positioning of the analyte monitor and/or to check one or more components of the analyte monitor, optical alert, sound alert.

In this case, an in situ test can also be repeatedly carried out, for example periodically, and each time the comparison leads to the conclusion that the analyte monitor could not be working properly, the user of the analyte monitor is informed about it and can proceed to the appropriate checks or modifications. A patient or an operator can as a consequence rely on the analyte monitor knowing that if the operation of the analyte monitor is incorrect, he will be promptly alerted.

In a particular embodiment of the process for in situ testing an analyte monitor based on photoacoustic or photothermal detection, if the absolute value of at least one correlation coefficient is lower than a threshold value, the process comprises between e) and f) at least one of:
g1) updating the window period and repeating d), e) and f) ;
g2) updating the irradiation configuration and repeating c), d), e) and f) ;
g3) imposing a forced modification on the target and/or the environment of the target and/or the analyte monitor and repeating b) c), d), e) and f).

Such an embodiment allows discriminating between no correlation due to the choice of the window period, no correlation due to the choice of the irradiation configuration, and no correlation due to an improper positioning of the sensor or no correlation due to a component failure. This allows as a consequence the user to implement the most appropriate corrective measures, for example to replace the analyte monitor only in case of a component failure and not in case of an improper choice of the window period.

In a particular embodiment of the process for in situ testing an analyte monitor based on photoacoustic or photothermal detection, the process can comprise both g1) and g2), and g2) can be implemented only if g1) has been repeated imax times, imax being a predetermined positive integer associated with the window period updating.

This embodiment makes it possible to limit the number of updates of the window period that allows one to conclude that the choice of the window period is not responsible for a no correlation observation. The maximum number of updates can be chosen according to the test period and/or the analyte and/or the target, in order to have a reasonable level of confidence in the decision with a reasonable calculation time.

In a particular embodiment of the process for in situ testing an analyte monitor based on photoacoustic or photothermal detection, the process comprises both g2) and g3), and g3) can be implemented only if g2) has been repeated jmax times, jmax being a predetermined positive integer associated with the irradiation configuration updating.

This embodiment makes it possible to limit the number of updates of the irradiation configuration that allows one to conclude that the choice of the irradiation configuration is not responsible for a no correlation observation. The maximum number of updates can be chosen according to the analyte and/or the target and a power consumption threshold, in order to have a reasonable level of confidence in the decision with an acceptable power consumption.

The invention also deals with a process for measuring an analyte level in the target with an analyte monitor based on photoacoustic or photothermal detection comprising the process for in situ testing the analyte monitor according to any of the preceding embodiments prior to at least one measurement step of the analyte level.

This allows to have a measurement process with high confidence level since the correct operation of the analyte monitor is controlled in situ prior to one or measurements of the analyte level, this control requiring no substantial modification of the analyte monitor and especially no additional component.

The invention further deals with an analyte monitor based on photoacoustic or photothermal detection for measuring an analyte level in a target comprising :
- an intensity-modulation device,
- a light emitter emitting an intensity-modulated light,
- a light emitter controller for controlling at least one modulation frequency at which said intensity-modulation device modulates the intensity of a light emitted by the light emitter,
- at least one detection cell comprising a sensor sensing directly or indirectly a thermal wave propagating out of the target in response to an irradiation,
- a processor module configured to receive and process sensor data from the at least one detection cell
characterized in that the processor module further comprises means adapted for :
i) setting an irradiation configuration comprising at least one triplet of irradiation parameters of a light source of the analyte monitor comprising a modulation frequency, a wavelength and an optical power;
ii) irradiating the target with the light source of the analyte monitor configured with the irradiation configuration at a plurality of irradiation time points and measuring for each time point at least an amplitude and a phase of an acoustic or thermal wave generated in response to the irradiation for at least one triplet of irradiation parameters of the irradiation configuration;
iii) calculating at least one correlation coefficient between the amplitude and the phase for at least one triplet of irradiation parameters of an irradiation configuration over a window period comprising at least two irradiation time points of with the light emitter;
iv) comparing at least one of the at least one correlation coefficient with at least one threshold value;
v) providing a test result based on the result of the comparison.

Last, the invention deals with a computer program comprising instructions to cause the analyte monitor according to the preceding embodiment to execute the in situ testing process of any of the preceding embodiments.

### BRIEF DESCRIPTION OF THE DRAWINGS

Embodiments of the invention will be described below, in relation to the following drawings :
Fig. 1 is a view of an exemplary analyte monitor based on photoacoustic detection on which the process according to the invention can be implemented.
Fig. 2 shows the results of a measurement campaign of the amplitude (plain line) and the phase (dotted line) of an acoustic wave generated in response to an irradiation at fmod = 100 Hz with a wavenumber of 1034 cm⁻¹ and with an optical power of 8 mW, the analyte monitor being on a patient.
Fig. 3 shows the results of a measurement campaign of the amplitude (plain line) and the phase (dotted line) of an acoustic wave generated in response to an irradiation at fmod = 100 Hz (on the left) and fmod = 600 Hz (on the right) with a wavenumber of 1034 cm⁻¹ and an optical power of 8 mW, the analyte monitor being on a patient.
Fig. 4 shows the results of a measurement campaign of the amplitude (plain line) and the phase (dotted line) of an acoustic wave generated in response to an irradiation at fmod = 100 Hz with a wavenumber of 1034 cm⁻¹ and an optical power of 8 mW, the analyte monitor being placed on a table.
Fig. 5 shows the results of a measurement campaign of the amplitude (plain line) and the phase (dotted line) of an acoustic wave generated in response to an irradiation at fmod = 100 Hz (on the left) and fmod = 600 Hz ( on the right) with a wavenumber of 1034 cm⁻¹ and an optical power of 8 mW, the analyte monitor being placed on a table.
Fig. 5b shows the results of a measurement campaign of the amplitude and the phase of an acoustic wave generated in response to an irradiation at fmod = 100 Hz with a wavenumber of 1034 cm-1, an optical power of 8 mW, the analyte monitor being successively :
   - placed on a table (from time = 1900 s to time = 3200 s),
   - placed on a patient's arm (from time = 3200 s to time = 5950 s),
   - and placed again on the table (from time =5950 s to time = 6950 s).
Fig. 6 shows the results of a measurement campaign of the amplitude (plain line) and the phase (dotted line) of an acoustic wave generated in response to an irradiation at fmod = 50 Hz, finod= 200 Hz, fmod = 500 Hz and fmod = 13 800 Hz with a wavenumber of 1034 cm⁻¹ and an optical power of 8 mW the analyte monitor being on a patient's arm.
Fig. 7 represents a flowchart of a first exemplary embodiment of the in situ testing process according to the invention.
Fig. 8 represents a flowchart of a second exemplary embodiment of the in situ testing process according to the invention.

On the drawings, the same reference signs show the same or similar objects.

### DETAILED DESCRIPTION

The invention deals with a process for testing in situ a non-invasive analyte monitor 1 based on photoacoustic or photothermal detection.

The non-invasive analyte monitor 1 may be used to analyse a target 2 and/or to measure a substance or analyte in a target 2, for example a human or an animal tissue, like skin.

For example, the substance or the analyte to be measured is blood glucose.

More specifically, a blood glucose value can be estimated based on a non-invasive interstitial glucose measurement.

The non-invasive analyte monitor 1 on which the process can be implemented can be portable or wearable. It comprises at least:
- an intensity-modulation device, a light emitter emitting an intensity-modulated light, a light emitter controller controlling at least one modulation frequency fmod at which said intensity-modulation device modulates the intensity of a light emitted by the light emitter,
- at least one detection cell 12 comprising a sensor sensing directly or indirectly a thermal wave propagating out of the target 2 in response to an irradiation,
- a processor module 13 configured to receive and process sensor data from the at least one detection cell 12.

In an exemplary embodiment, the detection cell 12 is a photoacoustic (PA) detection cell.

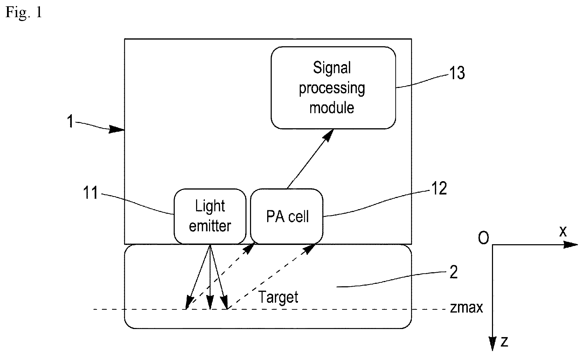

Fig. 1 shows a schematic diagram illustrating how photoacoustic measurement techniques may be used to obtain non-invasively an analyte sensor signal representative of an analyte concentration, for example a blood glucose concentration level of a user.

As can be seen on fig. 1, a light emitter block 11 may comprise a light emitter configured to emit a light beam towards a target 2. The target may be a patient's tissue, for example skin. The light incident on the target 2 penetrates into the target 2 and interacts with analyte molecules that are present inside the target 2, for example glucose molecules.

The analyte molecules are in consequence excited thermally.

As a result, a thermal wave propagates in the target 2, in particular toward the surface of the target 2 and, then into the medium surrounding the target 2, for example air.

In case a gaseous medium is surrounding the target 2, an acoustic wave associated with the thermal wave propagates in this gaseous medium surrounding the target 2.

Consequently, the thermal wave propagating in response to the irradiation of the target 2 can be directly or indirectly sensed.

In the first case, the thermal wave can be sensed with a thermal sensor included in the detection cell 12.

In the second case, one can for example use a photoacoustic sensor to sense the acoustic wave associated with the thermal wave in a gaseous medium surrounding the target 2.

The propagation of light and of the acoustic wave is schematically illustrated in fig. 1 through the use of respectively bold and dashed arrows.

In case the acoustic wave is sensed by a photoacoustic detection cell 12, this detection cell comprises a chamber filled with a gas (for example air) through which the acoustic wave propagates and one or more appropriate sensors placed in this chamber, for example opposite the target 2.

In an exemplary embodiment, the photoacoustic detection cell 12 comprises an electroacoustic sensor configured to convert the pressure of the acoustic wave into an electrical signal, such as a microphone. In an alternative exemplary embodiment, the photoacoustic detection cell 12 comprises a transducer, for example a piezoelectric transducer.

In exemplary embodiments, the electroacoustic sensor is operably connected to a signal processing module 13. The signal processing module 13 comprises an analog-to-digital converter configured to convert the analogic electrical signal from the electroacoustic sensor to a digital signal.

The signal processing module 13 optionally comprises an operational amplifier operably connected to the analog-to-digital converter and configured to further amplify the electronic signal derived from the acoustic response of the target 2.

In exemplary embodiments, the analog-to-digital converter is operably connected to a digital signal processor for processing of the digital signal.

In a particular embodiment, the light emitter emits an intensity-modulated laser beam at at least one particular wavelength towards the target 2.

In an exemplary embodiment, the light emitter is a light-emitting diode (LED).

In an alternative embodiment, the light emitter is a laser chip.

For example, the light emitter comprises a Quantum Cascade Laser (QCL) emitting in the mid-infrared region (MIR-QCL).

The light emitter block 11 also comprises the circuitry associated with the light emitter and a controller module configured to control the light emitter such that the intensity of the light emitted by the light emitter is modulated at a tunable intensity modulation frequency. The intensity modulation frequency is called fmod in the rest of the description.

The light emitter block 11 also comprises means to control at least one optical power P and at least one wavelength or equivalently one wavenumber characterising the light emitted by the light emitter.

The intensity modulation can be obtained by any known electric or mechanical means.

The light can be emitted continuously or in a pulsed manner.

In a particular embodiment, the light emitter controller module is configured to constrain the light emitter so as to emit light pulses. The pulses may have durations of about 100 ns to 500 ns per pulse, and a duty cycle for example of 1% to 20%, which corresponds to a pulse frequency of the order of 20 kHz to 2 MHz.

It must be understood that, in order to help understand the invention, a blood glucose sensor will often be described in the detailed examples below, but another analyte can be measured in the same way by merely adapting the wavenumber of the laser beam to the analyte to be monitored. The process and system described here can also be adapted to other targets 2 than skin. Whatever the analyte of interest or the target 2, the expected signal depends on numerous parameters making an accurate prediction of this expected signal complicated. In other words, the expected signal is not known and it is not possible to decide if the sensor is working correctly by comparing the measured signal and an expected in situ signal that would be calculated in advance.

Moreover, the fraction of the detected signal associated with the analyte of interest is often very low.

Last, the accuracy and the repeatability of the measurement depend on the positioning of the sensor 1 relative to the target 2. If the target 2 is moving or exposed to shocks or various contacts, the positioning of the analyte monitor 1 can be modified in an uncontrolled or undesired manner.

As a consequence, it is at first sight impossible, without requiring to an additional reference or an additional calibration unit, to check if the analyte monitor 1 is correctly operated, that is to say if there is no component failure or an incorrect position of the analyte monitor 1 or more generally that the detected signal can actually be associated with a value of the analyte of interest.

The analyte monitor 1 is configured to detect either a thermal wave or a pressure wave. In both cases, the detected signal is characterized for each triplet of irradiation parameters comprising a modulation frequency fmod, a wavelength λ (or equivalently a wavenumber v) and an optical power P by an amplitude A(fmod, λ, P) and a phase φ(fmod, λ, P).

The inventors have performed extensive experiments in particular in the case of an analyte monitor 1 based on photoacoustic detection for non invasively detecting an analyte such as interstitial glucose. They have steadily observed that when the analyte monitor 1 is correctly operated as defined above, the amplitude A(fmod, λ, P) and the phase φ(fmod, λ, P) for a given triplet of irradiation parameters are not independent from one another.

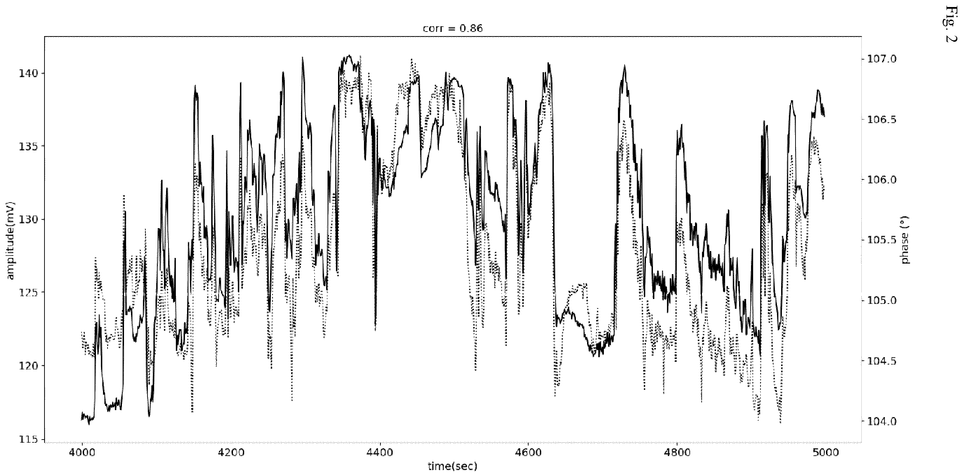

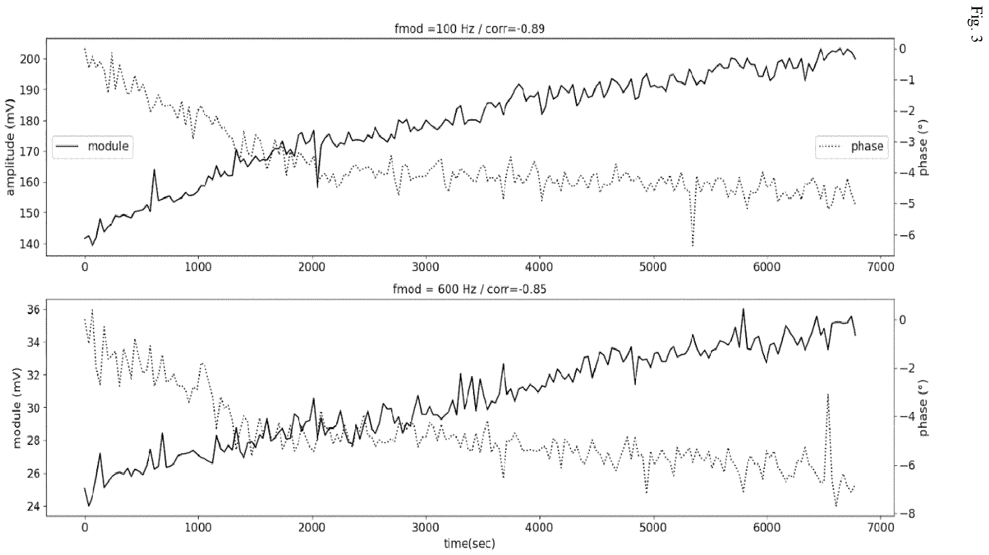

Depending at least on the target 2 and on the modulation frequency fmod, the amplitude A(fmod, λ, P) and the phase φ(fmod, λ, P) for a given triplet of irradiation parameters, and especially for a given modulation frequency and a given wavelength λ, can either be positively correlated as can be observed on fig. 2 or negatively correlated (or equivalently anti-correlated) as can be observed on fig. 3, but they are most often negatively correlated whatever the modulation frequency fmod.

In particular, in the case of fig. 3, R(finod = 100 Hz ,v = 1034 cm⁻¹, P = 8 mW) = - 0.89 and R(fmod = 600 Hz, v = 1034 cm⁻¹, P = 8 mW) = - 0.85 for measurements performed during about 2 hours, one measurement every 30 seconds.

In particular, in the case of fig. 2, R(finod = 100 Hz ,v = 1034 cm⁻¹, P = 8 mW) = + 0. for measurements performed during about 2 hours, one measurement every 30 seconds.

In the cases of fig. 2 and fig. 3, an analyte monitor 1 based on photoacoustic detection was positioned on the arm of a test patient. The correct positioning of the analyte monitor 1 was checked by the technician in charge of the experiment as well as all the components of the analyte monitor, so that there was no doubt concerning the fact that the analyte monitor 1 was correctly operated. A measurement campaign of the amplitude (plain line) and the phase (dotted line) of an acoustic wave generated in response to an irradiation at fmod = 100 Hz (and also fmod = 600 HZ for fig. 3) with a wavenumber of 1034 cm⁻¹ and with an optical power of 8 mW was then performed, with a measurement every 30 seconds during about 2 hours, the patients being different in the case of fig. 2 and in the case of fig. 3.

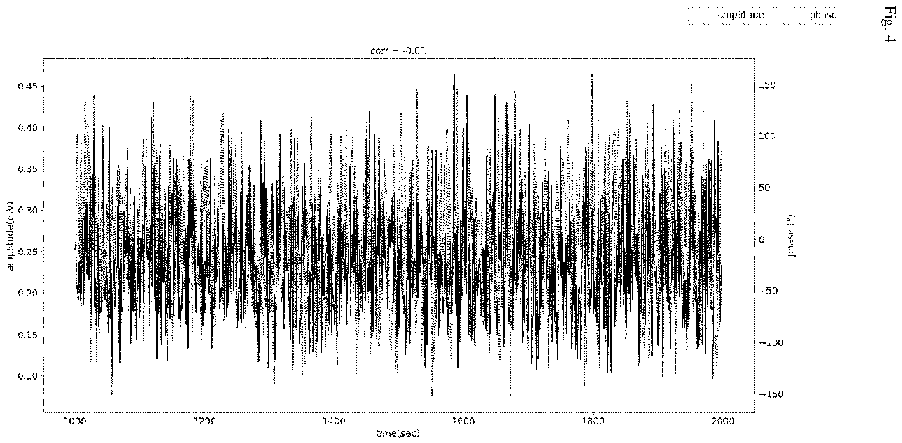

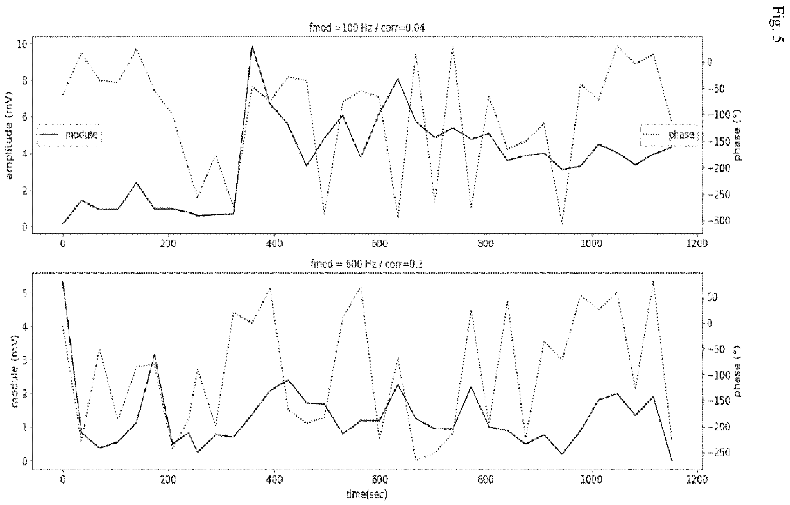

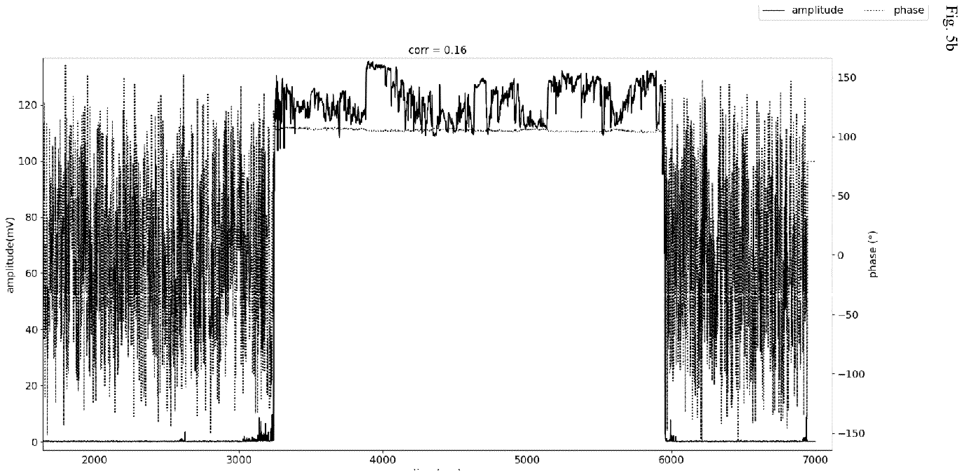

A comparative experiment was led in which the same analyte monitor 1 was just placed on a table in the same room right after or right before the experiment of fig. 2 or fig. 3. In each case, no correlation between the amplitude A(fmod, λ, P) and the phase φ(fmod, λ, P) of the detected signal at a given modulation frequency fmod and a given wavelength λ was observed. The experimental results are shown respectively on fig. 4 and fig. 5.

In particular, in the case of fig. 5, R(fmod = 100 Hz, v = 1034 cm⁻¹, P = 8 mW) = 0.05 and R(finod = 600 Hz, v = 1034 cm⁻¹, P = 8 mW) = 0.31 for measurements performed during about 2 hours, one measurement every 30 seconds, these coreelation coefficients being thus much lower in absolute value than in the case of fig. 3.

In the case of fig. 4, R(fmod = 100Hz, v = 1034 cm⁻¹, P = 8 mW) = 0.01, much lower than in the case of fig. 2 where R( fmod = 100 Hz, v = 1034 cm⁻¹, P = 8 mW) = 0.86.

No correlation means, as usually admitted, in the context of this document that the correlation coefficient R(fmod, λ, P) between the amplitude A(fmod, λ, P) and the phase φ(fmod, λ, P) for a given of triplet of irradiation parameters is too low to be significant, i.e. inferior to a threshold value. More precisely, the threshold value depends on the intrinsic noise of the system and on the quantification step of the analyte of interest. Typically, in the cases of fig. 2 to 5, the thershold value is between 0.5 and 0.8.

On fig. 3, one can see both the amplitude A(fmod, λ, P) and the phase φ(fmod, λ, P) of the detected signal globally increase during the experiment for an unknown reason, related to the physiological variations of the test patient during this experiment.

Fig. 5b gives a complete view of the experiments of fig. 2 and fig. 4 made in the following order: experimental conditions of fig. 4, immediately followed by experimental conditions of fig. 2, immediately followed by a repetition of the experimental conditions of fig. 4. The correlation coefficient between the amplitude and the phase at fmod =100 Hz with an optical power of 8 mW and a wavenumber of 1034 cm⁻¹ is equal to 0.16 over the whole window period but thiscorrelation coefficient is equal to 0.86 between 3200 s and 5950s. Hence, Fig. 5b shows clearly that when a significant signal is expected, there is a significant correlation between the amplitude A(fmod, λ, P) and the phase φ(fmod, λ, P) of the detected signal.

Another comparative experiment was led, in which a physiological variation was induced by the technician in the test patient and the consequences of this physiological variation were observed.

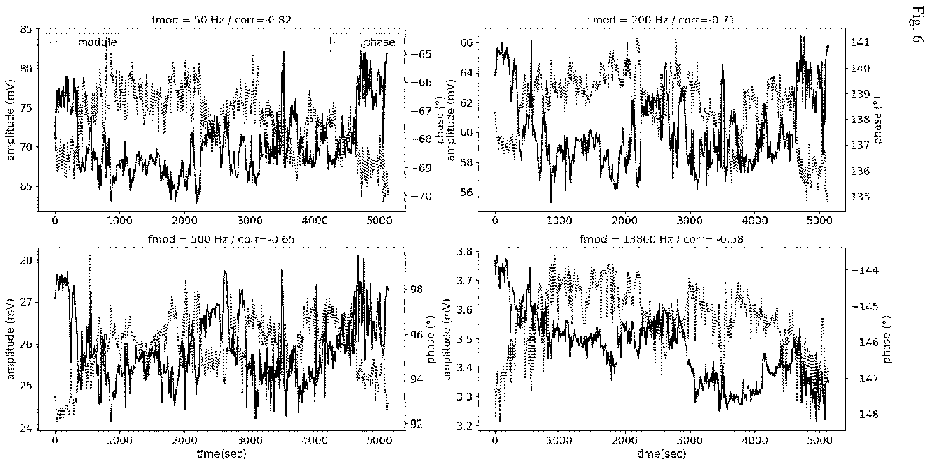

Namely, an occlusion experiment was carried out: the cuff maintaining the analyte monitor 1 on the arm of the patient was over-tightened so as to cause a change in blood distribution and a forced change in interstitial blood glucose. Part of the results of these experiments can be observed on fig. 6. One can state that, when a variation of the signal is expected due to a variation in the source of the signal, there are once again variations both of the amplitude A(fmod, λ, P) and the phase φ(fmod, λ, P), the positive or negative correlation amplitude A(fmod, λ, P) and the phase φ(fmod, λ, P) being maintained despite the modifications in the source. This observation is the same at all the modulation frequencies tested: R(finod=50Hz, v = 1034 cm⁻¹, P = 8 mW) = - 0.82 ; R(finod=200Hz, v = 1034 cm⁻¹, P = 8 mW) = - 0.71 ; R(fmod=500Hz, v = 1034 cm⁻¹, P = 8 mW) = - 0.65 ; R(fmod=13 800Hz, v = 1034 cm⁻¹, P = 8 mW) = - 0.58.

These experiments among numerous other ones convinced the inventors that when the amplitude A(fmod, λ, P) and the phase φ(fmod, λ, P) for a given triplet of irradiation parameters are correlated - either positively or negatively - over a long enough period, the detected signal can be considered as reliable and used for an analyte measurement whereas no correlation is an indication that something goes wrong in the measurement chain.

Obtaining such a correlation coefficient R(fmod, λ, P) between the amplitude A(fmod, λ, P) and the phase φ(fmod, λ, P) for a given triplet of irradiation parameters doesn't require any additional material since it requires exactly the same components as for an analyte measurement. As a consequence, the process for testing in situ a non-invasive analyte monitor 1 based on photoacoustic or photothermal detection that will be described hereafter solves the technical problem of in situ testing without increasing the size and/or the weight and/or the complexity of the analyte monitor 1. No additional integration issue is associated with the testing process according to the invention.

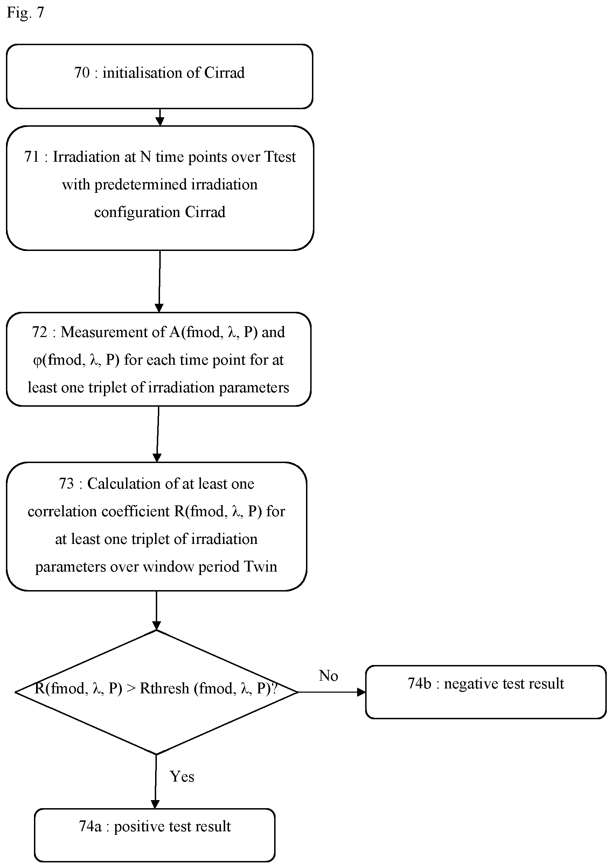

A particular embodiment of a flowchart representing the testing process according to the invention is presented on fig. 7.

The testing process according to the invention comprises:
1) Providing an analyte monitor 1 based on photoacoustic or photothermal detection.
2) Initialising an irradiation configuration Cirrad (Initialisation step 70).
3) At a current measurement position relative to a target, irradiating the target 2 with the predetermined irradiation configuration Cirrad (irradiation step 71) at a plurality of time points over a predetermined testing period Ttest,
   and measuring an amplitude A(fmod, λ, P) and a phase φ(fmod, λ, P) at at least one given triplet of irradiation parameters of a thermal or acoustic wave generated in response to the irradiation (measurement step 72) at the plurality of time points over the predetermined testing period Ttest.
4) Calculating at least one correlation coefficient R(fmod, λ, P) between the amplitude A(fmod, λ, P) and the phase φ(fmod, λ, P) at at least one of the at least one triplet of irradiation parameters over a window period Twin included in the predetermined testing period Ttest (step 73).
5) Comparing at least one of the at least one correlation coefficient R(fmod, λ, P) with at least one threshold value Rthresh.
6) Providing a test result based on the result of the comparison (step 74).

The testing process thus initially requires the acquisition of a series of measurements of the characteristics of the thermal or acoustic wave generated in response to a series of irradiations of the target 2 with a predetermined irradiation configuration Cirrad.

A predetermined irradiation configuration Cirrad of the light emitter 11 of the analyte monitor 1 comprises at least one triplet of irradiation parameters that comprises a modulation frequency fmod of the intensity of the laser, a wavelength λ, and a surface power density SPD or an optical power P.

The amplitude A(fmod, λ, P) and the phase φ(fmod, λ, P) (or equivalently the amplitude A(fmod, λ, SPD) and the phase φ(fmod λ, SPD)) are measured in situ, at the current measurement position relative to the target 2 for at least one given triplet of irradiation parameters comprised in Cirrad for every irradiation time point.

The current measurement position relative to the target 2 should be the nominal measurement position relative to the target 2, which can depend on the target 2 and on the analyte to be measured. For example, in case of a continuous glucose monitor, this nominal position can be at a particular location of the arm of a patient, the detection cell being in direct contact with the skin of the patient. In this case, if the patient moves abruptly or doesn't install carefully the glucose monitor, the current measurement position relative to the patient (the target 2 in this case) is different from the nominal measurement position.

The predetermined irradiation configuration Cirrad can comprise one or more triplets of irradiation parameters, depending among others on the desired confidence level of the test result and on the authorized power consumption of the analyte monitor 1 during the testing process. The one or more triplets of irradiation parameters, and especially the one or more modulation frequencies can be chosen according to the structure of the target 2 and/or to the composition of the target 2.

The one or more wavelengths of the predetermined irradiation configuration Cirrad can be chosen among others according to the analyte of interest.

The one or more optical powers P or surface power densities SPD of the irradiation configuration Cirrad can also be set among others depending for example in an authorized power consumption during the testing process and/or a confidence level threshold for this testing process.

In case of a glucose monitor, the initial predetermined irradiation configuration Cirrad chosen at initialization step 70 can be a "mean configuration" that is to say an irradiation configuration Cirrad that is the more likely to generate a reliable acoustic or thermal signal if the analyte monitor 1 is correctly operated for any patient of a given group of patients and/or for the physiological current event (sport, sleep, meal, etc.).

The time points corresponding to the series of irradiations can be regularly spaced (periodic irradiation) or not, and the series of irradiations extends over a predetermined period of time called Ttest or testing period.

The testing period Ttest can be chosen according to the target 2 and/or to the analyte of interest. As a general rule, Ttest is long enough for the correlation coefficient to be significant.

For example, in case of a continuous glucose monitor, the glycemia can change at a rate as high as 1 to 5 mg/dL/min. Depending on the desired detection limit, Ttest can be of the order of 1 to 10 minutes with at least 5 times points distributed over Ttest.

The number of irradiation time points N and their spacing or distribution - and consequently Ttest - can also be chosen according to the target 2 and/or to the analyte of interest.

Once the amplitude A(fmod, λ, P) and the phase φ(fmod λ, P) for at least one triplet of irradiation parameters are measured for each irradiation time point, the processing unit 13 of the analyte monitor 1 performs a calculation of a correlation coefficient R(fmod, λ, P) between the amplitude A(fmod, λ, P) and the phase φ(fmod, λ, P) for part of or each of the triplets of irradiation parameters of the irradiation configuration Cirrad for each time point comprised in a window period Twin included in the testing period Ttest.

By default, Twin can be equal to Ttest.

If an only triplet of irradiation parameters is exploited, the absolute value of the correlation coefficient R(fmod, λ, P) is then compared to a predetermined threshold value Rthres(finod, λ, P) chosen according to the given modulation frequency fmod, the given wavelength λ and the given optical power P.

The predetermined threshold value can be the same for all the modulation frequencies or depend on the modulation frequency. The predetermined threshold value can be the same for all the optical powers or depend on the optical power. The predetermined threshold value can be the same for all the wavelengthes or depend on the wavelength.

The predetermined threshold value Rthresh(fmod, λ, P) can be more than 0.5 ; more than 0.6 ; more than 0.7 ; more than 0.8 ; more than 0.9 and it can depend on a desired confidence level of the testing process for a given analyte for a given type of target 2.

If the irradiation configuration Cirrad comprises several triplets of irradiation parameters (modulation frequency fmod, wavelength λ, optical power P), a mean value Rmean of the absolute values of the correlation coefficients R(λ, fmod, P) for part or for all these pairs can be calculated with the processing unit 13 to enhance the accuracy of the testing process.

In another embodiment, the comparison with a threshold value can be based on a distance D between the tuple formed by all the absolute values of the correlation coefficients R(λ, fmod, P) for all the triplets of irradiation parameters exploited and a reference tuple.

In a particular embodiment, the maximum of the absolute values of the correlation coefficients R(λ, fmod, P) for all these triplets of irradiation parameters can be compared to a threshold value.

Last, a test result is provided by the processing unit 13 based on the result of the comparison.

If the absolute value of the correlation coefficient (or the mean value Rmean or the distance D) is higher than the predetermined threshold value, the operation of the analyte monitor 1 is correct and the analyte monitor 1 can be used for a following time period for an analyte measurement. The test result is a positive test result 74a.

A positive test result 74a can thus consist in an instruction for the analyte monitor 1 to start or to resume a measurement sequence. A positive test result 74a can also consist in an instruction for the analyte monitor 1 to display a message on an user interface or any equivalent comprising information that the operation of the analyte monitor has been validated.

If the absolute value of the correlation coefficient (or the mean value Rmean or the distance D) is lower than the predetermined threshold value, the operation of the analyte monitor 1 could be incorrect.

In a first embodiment, the test result is thus a negative test result 74b as represented on fig. 7.

A negative test result 74b can consist in an instruction for the analyte monitor 1 to display an warning message on a user interface. For example, the user could be prompted to check the positioning or one or more components of the analyte monitor 1.

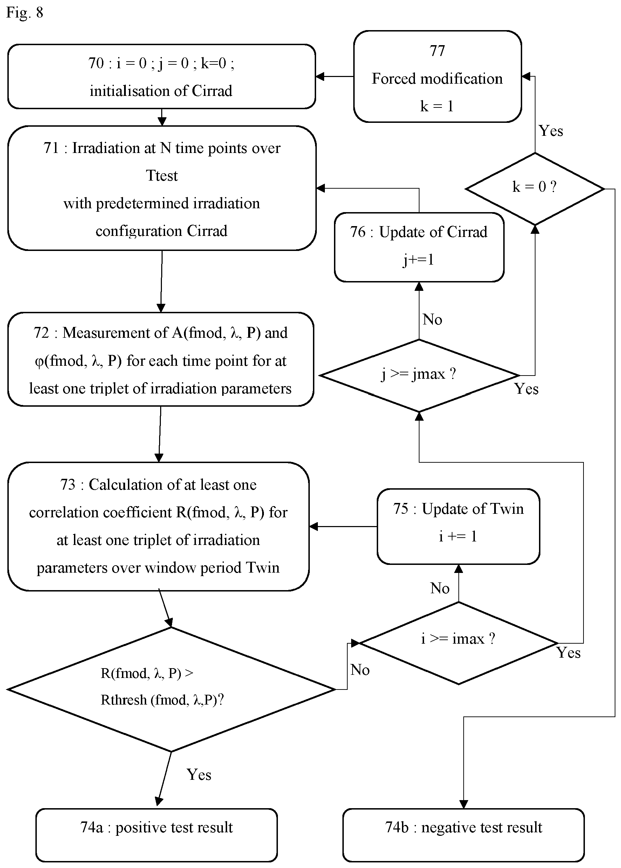

In another embodiment represented on fig. 8, before deciding that the test result is negative, one or more optional additional steps can be carried out.

The first optional additional step is an update step 75 of the window period Twin.

Indeed, even if no correlation has been observed first, a correlation could be observed:
- either on a shorter window period without being observed in the initial window period, depending on the characteristic times of the different phenomena involved : a correlation could exist over parts of the initial window period but not over the initial window period as a whole, as can be seen on fig. 5b (correlation coefficient equal to 0.16 over the whole window period but correlation coefficient equal to 0.86 between 3200 s and 5950s)
- or on an offset window period relative to the initial window period, due for example to edge effects.

In a particular embodiment, the calculation of the one or more correlation coefficients R(finod, λ, P) can be iterated with a sliding window period Twin in order to ensure that there is no missed correlation due to the choice of the starting point of the window period Twin.

If the current window period Twin is less than the testing period Ttest, updating the window period can consist in changing the starting point of Twin, unless all segments of Ttest having a duration equal to Twin have been tried.

Updating the window period Twin can consist in shortening the current window period Twin by subtracting a predetermined duration to it or by multiplying it by a predetermined ratio.

In another embodiment, the update of the window period Twin can consist in a shortening of the window period. In both cases, if the correlation coefficient (or the mean value Rmean or the distance D) is lower than the predetermined threshold value, the processor 13 receives an instruction for the analyte monitor 1 to update the window period Twin (step 75) and repeat step 73: calculate at least one correlation coefficient R(fmod, λ, P) between the amplitude A(fmod, λ, P) and the phase φ(fmod, λ, P) for the at least one triplet of irradiation parameters over the new window period Twin, as well as the following steps.

A counter i of the window period updates can be initialized at the initialisation step 70 and incremented at each window period update 75, so that the number of window period updates can be limited with a maximum value imax as shown on fig. 8.

The second optional additional step is an update step 76 of the irradiation configuration Cirrad. Indeed, even if no correlation has been observed first, a correlation could be observed with another irradiation configuration. For example, in the case of a glucose monitor 1 if the default irradiation configuration Cirrad at the irradiation step 70 is a "mean irradiation configuration", another irradiation configuration Cirrad could be chosen with regard to a patient history ensuring with higher confidence level that if operation of the analyte monitor 1 is correct, a correlation should be observed.

Once the irradiation configuration Cirrad has been updated, the irradiation step 71 and the following steps are repeated as shown on fig. 8.

The update step 76 of the irradiation configuration Cirrad can be carried without an update step 75 of the window period, or after the counter i for the updates of the window period Twin if any has reached its maximum value imax as represented on fig. 8.

A counter j of the irradiation configuration updates can be initialized at the initialisation step 70 and incremented at each irradiation configuration update 76, so that the number of irradiation configuration updates can be limited with a maximum value jmax as shown on fig. 8.

The third optional additional step is a step 77 comprising imposing a change to the target 2 and/or to its environment and/or to the analyte monitor 1.

Indeed, even if no correlation has been observed first, this could be due to the fact that the ratio noise signal is too high without any failure of the analyte monitor 1 or improper operation of this analyte monitor 1. In order to rule out this hypothesis, a modification in the target 2 or its environment could be forced so as to cause a modification in the signal that should result in a subsequent observable correlation.

In the glucose monitor example, the modification could be a tightening of the cuff either automatically or by the patient such that, if operation of the analyte monitor 1 is correct, the impact of the occlusion is sufficient for a subsequent correlation between amplitude and phase at one or more modulation frequencies fmod and one or more wavelengths λ (even not specific to glucose, for example specific to water) to be observed.

Once the forced modification has been implemented, the irradiation configuration Cirrad is initialised accordingly (step 70) and the irradiation step 71 and the following steps are repeated as shown on fig. 8.

The forced modification step 77 can be carried without an update step 75 of the window period and/or an update step 76 of the irradiation configuration, or after one or both of the counter i for the updates of the window period Twin and the counter j for the updates of the irradiation configuration if any have reached their respective maximum value imax (resp. jmax) as represented on fig. 8.

A counter k of the forced modification can be initialized at the initialisation step 70 and incremented at each forced modification, so that the number of irradiation configuration updates can be limited with a maximum value kmax. In the embodiment of fig.8, kmax = 1 so that an only forced modification is provoked if needed, but several different forced modifications could be considered on the same principle.

As non limiting examples in case of a glucose monitor, a forced modification could be:
- a re-positioning of the analyte monitor 1 by the user,
- a specific movement of the arm on which the analyte monitor 1 is positioned,
- a controlled occlusion,
- a lowering of the temperature of the hand (placed in contact with cold water for example),
- a controlled glucose intake
- etc,

The process for in situ testing an analyte monitor 1 can comprise one or more of the three optional additional steps. These additional steps are optional in that they are not mandatory for the process to be operated but they contribute to ensure that a negative result 74b is not a false negative result 74b corresponding to an intrinsic lack of correlation or, in other words, to no correlation without a cause in the operation in the analyte monitor 1: the additional steps contribute to increase the confidence level of the testing process according to the invention. The invention also deals with an analyte monitor 1 comprising a processor 13 configured to execute the process for in situ testing the analyte monitor 1, and with a computer program comprising the instructions for causing an analyte monitor 1 to execute the steps of the process for in situ testing the analyte monitor 1.

### LIST OF THE REFERENCE SIGNS

1 : analyte monitor
11 : light emitter block
12 : detection cell
13 : processor module
2 : target

## Claims

1. Process for in situ testing an analyte monitor (1) based on photoacoustic or photothermal detection comprising:
a) providing an analyte monitor (1) based on photoacoustic or photothermal detection at a measurement position relative to a target (2);
b) setting an irradiation configuration (Cirrad) of a light source of the analyte monitor (1) comprising at least one triplet of irradiation parameters comprising a modulation frequency (fmod), a wavelength λ and an optical power P;
c) irradiating the target (2) with the light source of the analyte monitor (1) configured with the irradiation configuration (Cirrad) at a plurality of irradiation time points and measuring for each time point at least an amplitude (A(fmod, λ, P)) and a phase (φ(fmod, λ, P)) of an acoustic or thermal wave generated in response to the irradiation for at least one triplet of irradiation parameters (fmod, λ, P) of the irradiation configuration (Cirrad);
**characterized in that** the process comprises the following steps:
d) calculating with a processor (13) of the analyte monitor (1) at least one correlation coefficient (R(fmod, λ, P)) between the amplitude (A(fmod, λ, P)) and the phase (φ(fmod, λ, P)) for at least one triplet of irradiation parameters (fmod, λ, P) of the irradiation configuration (Cirrad) over a window period (Twin) comprising at least two irradiation time points of said plurality of irradiation time points;
e) comparing with the processor (13) of the analyte monitor (1) at least one of the at least one correlation coefficient (R(fmod, λ, P)) with at least one threshold value (Rthresh);
f) providing with a processor (13) of the analyte monitor (1) a test result based on the result of the comparison.

2. Process for in situ testing an analyte monitor (1) based on photoacoustic or photothermal detection according to claim 1 in which if the absolute value of at least one correlation coefficient (R(fmod, λ, P)) is higher than a threshold value (Rthresh), the test result is an authorisation to proceed with at least one subsequent measurement with the analyte monitor (1).

3. Process for in situ testing an analyte monitor (1) based on photoacoustic or photothermal detection according to claim 1 or claim 2 in which if the absolute value of at least one correlation coefficient (R(fmod, λ, P)) is lower than a threshold value (Rthresh), the test result comprises displaying an alert chosen in the list: message on a user interface of the analyte monitor (1) or by emailing to check the positioning of the analyte monitor (1) and/or to check one or more components of the analyte monitor (1), optical alert, sound alert.

4. Process for in situ testing an analyte monitor (1) based on photoacoustic or photothermal detection according to any of claims 1-3 comprising, if the absolute value of at least one correlation coefficient (R(fmod, λ, P)) is lower than a threshold value (Rthresh), between e) and f) at least one of:
g1) updating the window period (Twin) and repeating d), e) and f) ;
g2) updating the irradiation configuration (Cirrad) and repeating c), d), e) and f) ;
g3) imposing a forced modification on the target (2) and/or the environment of the target (2) and/or the analyte monitor (1) and repeating b) c), d), e) and f).

5. Process for in situ testing an analyte monitor (1) based on photoacoustic or photothermal detection according to claim 4 comprising both g1) and g2), and in which g2) is implemented only if g1) has been repeated imax times, imax being a predetermined positive integer associated with the window period updating.

6. Process for in situ testing an analyte monitor (1) based on photoacoustic or photothermal detection according to claim 4 or claim 5 comprising both g2) and g3), and in which g3) is implemented only if g2) has been repeated jmax times, jmax being a predetermined positive integer associated with the irradiation configuration updating.

7. Process for measuring an analyte level in the target (2) with an analyte monitor (1) based on photoacoustic or photothermal detection comprising the process for in situ testing the analyte monitor (1) according to any of claims 1 to 6 prior to at least one measurement step of the analyte level.

8. Analyte monitor (1) based on photoacoustic or photothermal detection for measuring an analyte level in a target (2) comprising :
- an intensity-modulation device,
- a light emitter emitting an intensity-modulated light,
- a light emitter controller for controlling at least one modulation frequency (fmod) at which said intensity-modulation device modulates the intensity of a light emitted by the light emitter,
- at least one detection cell (12) comprising a sensor sensing directly or indirectly a thermal wave propagating out of the target (2) in response to an irradiation,
- a processor module (13) configured to receive and process sensor data from the at least one detection cell (12), wherein the processor module (13) further comprises means adapted for :
i) setting an irradiation configuration (Cirrad) comprising at least one triplet of irradiation parameters of a light source of the analyte monitor (1) comprising a modulation frequency (fmod), a wavelength (λ) and an optical power (P);
ii) irradiating the target (2) with the light source of the analyte monitor (1) configured with the irradiation configuration (Cirrad) at a plurality of irradiation time points and measuring for each time point at least an amplitude (A(fmod, λ, P)) and a phase (φ(fmod, λ, P)) of an acoustic or thermal wave generated in response to the irradiation for at least one triplet of irradiation parameters (fmod, λ, P) of the irradiation configuration (Cirrad);
**characterized by**:
iii) calculating at least one correlation coefficient (R(fmod, λ, P)) between the amplitude (A(fmod, λ, P)) and the phase (φ(fmod, λ, P)) for at least one triplet of irradiation parameters (fmod, λ, P) of an irradiation configuration (Cirrad) over a window period (Twin) comprising at least two irradiation time points of with the light emitter;
iv) comparing at least one of the at least one correlation coefficient (R(fmod, λ, P)) with at least one threshold value (Rthresh);
v) providing a test result based on the result of the comparison.

9. Computer program comprising instructions to cause the analyte monitor (1) of claim 8 to acquire measurement data obtained at step c) of claim 1 and execute steps d)-f) of the in situ testing process of claim 1 or the in situ testing process of any of claims 2 to 6.

## Patentansprüche

1. Verfahren zur In-situ-Prüfung eines Analytenmonitors (1), der auf photoakustischer oder photothermischer Detektion basiert, umfassend:
a) Bereitstellen eines Analytenmonitors (1), der auf photoakustischer oder photothermischer Detektion basiert, an einer Messposition in Bezug auf ein Ziel (2);
b) Einstellen einer Irradiationskonfiguration (Cirrad) einer Lichtquelle des Analytenmonitors (1), die mindestens ein Tripel von Irradiationsparametern umfasst, bestehend aus einer Modulationsfrequenz (fmod), einer Wellenlänge A und einer optischen Leistung P;
c) Bestrahlung des Ziels (2) mit der gemäß der Irradiationskonfiguration (Cirrad) konfigurierten Lichtquelle des Analytenmonitors (1) zu einer Vielzahl von Bestrahlungszeitpunkten und Messung für jeden Zeitpunkt mindestens einer Amplitude (A(fmod, λ, P)) sowie einer Phase (φ(fmod, λ, P)) einer durch die Bestrahlung erzeugten akustischen oder thermischen Welle für mindestens ein Tripel von Irradiationsparametern (fmod, λ, P) der Irradiationskonfiguration (Cirrad);
**dadurch gekennzeichnet, dass** das Verfahren die folgenden Schritte umfasst:
d) Berechnen mit einem Prozessor (13) des Analytenmonitors (1) mindestens eines Korrelationskoeffizienten (R(fmod, λ, P)) zwischen der Amplitude (A(fmod, λ, P)) und der Phase (φ(fmod, λ, P)) für mindestens ein Tripel von Irradiationsparametern (fmod, λ, P) der Irradiationskonfiguration (Cirrad) über ein Zeitfenster (Twin), das mindestens zwei Bestrahlungszeitpunkte der genannten Vielzahl von Bestrahlungszeitpunkten umfasst;
e) Vergleichen mittels des Prozessors (13) des Analytenmonitors (1) mindestens eines der Korrelationskoeffizienten (R(fmod, λ, P)) mit mindestens einem Schwellenwert (Rthresh);
f) Bereitstellen mittels eines Prozessors (13) des Analytenmonitors (1) eines Testergebnisses basierend auf dem Ergebnis des Vergleichs.

2. Verfahren zur In-situ-Prüfung eines Analytenmonitors (1) auf Basis photoakustischer oder photothermischer Detektion gemäß Anspruch 1, wobei, wenn der Absolutwert von mindestens einem Korrelationskoeffizienten (R(fmod, λ, P)) größer als ein Schwellenwert (Rthresh) ist, das Testergebnis eine Freigabe zur Durchführung von mindestens einer nachfolgenden Messung mit dem Analytenmonitor (1) darstellt.

3. Verfahren zur In-situ-Prüfung eines Analytenmonitors (1) auf Basis photoakustischer oder photothermischer Detektion gemäß Anspruch 1 oder Anspruch 2, wobei, wenn der Absolutwert von mindestens einem Korrelationskoeffizienten (R(fmod, λ, P)) kleiner als ein Schwellenwert (Rthresh) ist, das Testergebnis das Anzeigen einer Alarmmeldung aus der Liste umfasst: Meldung auf einer Benutzeroberfläche des Analytenmonitors (1) oder per E-Mail, um die Positionierung des Analytenmonitors (1) zu überprüfen und/oder ein oder mehrere Bauteile des Analytenmonitors (1) zu überprüfen, optische Warnung, akustische Warnung.

4. Verfahren zur In-situ-Prüfung eines Analytenmonitors (1), der auf photoakustischer oder photothermischer Detektion beruht, gemäß einem der Ansprüche 1-3, wobei, falls der Absolutwert mindestens eines Korrelationskoeffizienten (R(fmod, λ, P)) kleiner ist als ein Schwellenwert (Rthresh), zwischen e) und f) mindestens eines der folgenden:
gl) Aktualisierung des Zeitfensters (Twin) und Wiederholung von d), e) und f);
g2) Aktualisierung der Irradiationskonfiguration (Cirrad) und Wiederholung von c), d), e) und f);
g3) Erzwingung einer Änderung am Ziel (2) und/oder an der Umgebung des Ziels (2) und/oder am Analytenmonitor (1) und Wiederholung von b), c), d), e) und f).

5. Verfahren zur In-situ-Prüfung eines Analytenmonitors (1), der auf photoakustischer oder photothermischer Detektion beruht, gemäß Anspruch 4, das sowohl gl) als auch g2) umfasst, wobei g2) nur dann durchgeführt wird, wenn g1) imax-mal wiederholt wurde, wobei imax eine vordefinierte positive ganze Zahl ist, die mit der Aktualisierung des Zeitfensters verbunden ist.

6. Verfahren zur In-situ-Prüfung eines Analytenmonitors (1), der auf photoakustischer oder photothermischer Detektion beruht, gemäß Anspruch 4 oder Anspruch 5, das sowohl g2) als auch g3) umfasst, wobei g3) nur dann durchgeführt wird, wenn g2) jmax-mal wiederholt wurde, wobei jmax eine vordefinierte positive ganze Zahl ist, die mit der Aktualisierung der Irradiationskonfiguration verbunden ist.

7. Verfahren zur Messung einer Analytenkonzentration im Ziel (2) mit einem Analytenmonitor (1), der auf photoakustischer oder photothermischer Detektion beruht, umfassend das Verfahren zur In-situ-Prüfung des Analytenmonitors (1) gemäß einem der Ansprüche 1 bis 6 vor mindestens einem Messschritt der Analytenkonzentration.

8. Analytenmonitor (1) basierend auf photoakustischer oder photothermischer Detektion zur Messung einer Analytenkonzentration in einem Ziel (2), umfassend:
- eine Intensitätsmodulationsvorrichtung,
- ein Lichtemitter, der intensitätsmoduliertes Licht emittiert,
- ein Lichtemitter-Controller zur Steuerung mindestens einer Modulationsfrequenz (fmod), bei der die genannte Intensitätsmodulationsvorrichtung die Intensität des vom Lichtemitter ausgestrahlten Lichts moduliert,
- mindestens eine Detektionszelle (12), die einen Sensor umfasst, der direkt oder indirekt eine als Reaktion auf eine Bestrahlung aus dem Ziel (2) ausgehende thermische Welle erfasst,
- ein Prozessormodul (13), das zum Empfangen und Verarbeiten von Sensordaten der mindestens einen Detektionszelle (12) konfiguriert ist,
Wobei das Prozessormodul (13) ferner Mittel aufweist, die dazu eingerichtet sind:
i) Festlegung einer Irradiationskonfiguration (Cirrad), die mindestens ein Tripel von Bestrahlungsparametern einer Lichtquelle des Analytenmonitors (1) umfasst, nämlich eine Modulationsfrequenz (fmod), eine Wellenlänge (1) und eine optische Leistung (P);
ii) Bestrahlung des Ziels (2) mit der gemäß der Irradiationskonfiguration (Cirrad) konfigurierten Lichtquelle des Analytenmonitors (1) zu mehreren Bestrahlungszeitpunkten und Messung für jeden Zeitpunkt mindestens einer Amplitude (A(fmod, λ, P)) und einer Phase (φ(fmod, λ, P)) einer akustischen oder thermischen Welle, die als Reaktion auf die Bestrahlung erzeugt wird, für mindestens ein Tripel von Bestrahlungsparametern (fmod, λ, P) der Irradiationskonfiguration (Cirrad);
**gekennzeichnet durch**:
iii) Berechnung von mindestens einem Korrelationskoeffizienten (R(fmod, λ, P)) zwischen der Amplitude (A(fmod, λ, P)) und der Phase (φ(fmod, λ, P)) für mindestens ein Tripel von Bestrahlungsparametern (fmod, λ, P) einer Irradiationskonfiguration (Cirrad) über ein Zeitfenster (Twin), das mindestens zwei Bestrahlungszeitpunkte mit dem Lichtemitter umfasst;
iv) Vergleich mindestens eines der mindestens einen Korrelationskoeffizienten (R(fmod, λ, P)) mit mindestens einem Schwellenwert (Rthresh);
v) Bereitstellung eines Testergebnisses basierend auf dem Ergebnis des Vergleichs.

9. Computerprogramm mit Anweisungen, die den Analytenmonitor (1) gemäß Anspruch 8 veranlassen, Messdaten zu erfassen, die in Schritt c) von Anspruch 1 erhalten wurden, und
die Schritte d)-f) des In-situ-Prüfverfahrens gemäß Anspruch 1 oder des In-situ-Prüfverfahrens gemäß einem der Ansprüche 2 bis 6 auszuführen.

## Revendications

1. Procédé de test in situ d'un dispositif de surveillance d'analyte (1) basé sur la détection photoacoustique ou photothermique, comprenant :
a) la mise en place d'un dispositif de surveillance d'analyte (1) basé sur la détection photoacoustique ou photothermique en un point de mesure par rapport à une cible (2) ;
b) la configuration d'irradiation (Cirrad) de la source lumineuse du dispositif de surveillance d'analyte (1) comprenant au moins un triplet de paramètres d'irradiation : une fréquence de modulation (fmod), une longueur d'onde λ et une puissance optique P ;
c) l'irradiation d'une cible (2) avec la source lumineuse du dispositif de surveillance d'analyte (1) configurée selon le mode d'irradiation (Cirrad) à plusieurs instants d'irradiation et la mesure pour chaque instant d'au moins une amplitude (A(fmod, λ, P)) et une phase (ϕ(fmod, λ, P)) d'une onde acoustique ou thermique générée en réponse à l'irradiation pour au moins un triplet de paramètres d'irradiation (fmod, λ, P) de la configuration d'irradiation (Cirrad) ;
le procédé se **caractérise par** les étapes suivantes :
d) le calcul, à l'aide d'un processeur (13) du dispositif de surveillance d'analyte (1), d'au moins un coefficient de corrélation (R(fmod, λ, P)) entre l'amplitude (A(fmod, λ, P)) et la phase (ϕ(fmod, λ, P)) pour au moins un triplet de paramètres d'irradiation (fmod, λ, P) de la configuration d'irradiation (Cirrad) sur une période de fenêtre (Twin) comprenant au moins deux instants d'irradiation parmi la pluralité d'instants d'irradiation ;
e) la comparaison, à l'aide du processeur (13) du dispositif de surveillance d'analyte (1), d'au moins un des coefficients de corrélation (R(fmod, λ, P)) avec au moins une valeur seuil (Rthresh) ;
f) la fourniture, par le processeur (13) du dispositif de surveillance d'analyte (1), d'un résultat de test basé sur le résultat de la comparaison.

2. Procédé de test in situ d'un dispositif de surveillance d'analyte (1) basé sur la détection photoacoustique ou photothermique selon la revendication 1, dans lequel si la valeur absolue d'au moins un coefficient de corrélation (R(fmod, λ, P)) est supérieure à une valeur seuil (Rₜₕᵣₑₛₕ), le résultat du test autorise la réalisation d'au moins une mesure ultérieure avec le dispositif de surveillance d'analyte (1).

3. Procédé de test in situ d'un dispositif de surveillance d'analyte (1) basé sur la détection photoacoustique ou photothermique selon la revendication 1 ou 2, dans lequel si la valeur absolue d'au moins un coefficient de corrélation (R(fmod, λ, P)) est inférieure à une valeur seuil (Rₜₕᵣₑₛₕ), le résultat du test consiste à afficher une alerte choisie parmi les suivantes : message sur l'interface utilisateur du dispositif de surveillance d'analyte (1) ou envoi d'un courriel pour vérifier le positionnement du dispositif de surveillance d'analyte (1) et/ou un ou plusieurs de ses composants ; alerte optique ; alerte sonore.

4. Procédé de test in situ d'un dispositif de surveillance d'analyte (1) basé sur la détection photoacoustique ou photothermique selon l'une quelconque des revendications 1 à 3, comprenant, si la valeur absolue d'au moins un coefficient de corrélation (R(fmod, λ, P)) est inférieure à une valeur seuil (Rthresh), entre e) et f), au moins une des opérations suivantes :
g1) mise à jour de la période de la fenêtre (Twin) et répétition des étapes d), e) et f) ;
g2) mise à jour de la configuration d'irradiation (Cirrad) et répétition des étapes c), d), e) et f) ;
g3) application d'une modification forcée à la cible (2) et/ou à son environnement et/ou au dispositif de surveillance d'analyte (1), et répétition des étapes b), c), d), e) et f).

5. Procédé de test in situ d'un dispositif de surveillance d'analyte (1) basé sur la détection photoacoustique ou photothermique selon la revendication 4, comprenant à la fois g1) et g2), et dans lequel g2) n'est mis en œuvre que si g1) a été répété imax fois, imax étant un entier positif prédéterminé associé à la mise à jour de la période de fenêtre.

6. Procédé de test in situ d'un dispositif de surveillance d'analyte (1) basé sur la détection photoacoustique ou photothermique selon la revendication 4 ou 5, comprenant à la fois g2) et g3), et dans lequel g3) n'est mis en œuvre que si g2) a été répété jmax fois, jmax étant un entier positif prédéterminé associé à la mise à jour de la configuration d'irradiation.

7. Procédé de mesure du niveau d'un analyte dans la cible (2) avec un dispositif de surveillance d'analyte (1) basé sur la détection photoacoustique ou photothermique, comprenant le procédé de test in situ du dispositif de surveillance d'analyte (1) selon l'une quelconque des revendications 1 à 6 avant au moins une étape de mesure du niveau d'analyte.

8. Moniteur d'analyte (1) basé sur la détection photoacoustique ou photothermique pour la mesure du niveau d'un analyte dans une cible (2), comprenant :
- un dispositif de modulation d'intensité,
- un émetteur de lumière émettant une lumière à intensité modulée,
- un contrôleur d'émetteur de lumière permettant de contrôler au moins une fréquence de modulation (fmod) à laquelle ledit dispositif de modulation d'intensité module l'intensité de la lumière émise par l'émetteur,
- au moins une cellule de détection (12) comprenant un capteur détectant directement ou indirectement une onde thermique se propageant hors de la cible (2) en réponse à une irradiation,
- un module de traitement (13) configuré pour recevoir et traiter les données du capteur provenant de la ou des cellules de détection (12),
Dans lequel le module de traitement (13) comprend en outre des moyens adaptés pour :
i) définir une configuration d'irradiation (Cirrad) comprenant au moins un triplet de paramètres d'irradiation d'une source lumineuse du dispositif de surveillance d'analyte (1), comprenant une fréquence de modulation (fmod), une longueur d'onde (λ) et un paramètre optique puissance (P) ;
ii) irradier la cible (2) avec la source lumineuse du dispositif de surveillance d'analyte (1) configurée selon la configuration d'irradiation (Cirrad) à plusieurs instants d'irradiation et mesure, pour chaque instant, au moins une amplitude (A(fmod, λ, P)) et une phase (ϕ(fmod, λ, P)) d'une onde acoustique ou thermique générée en réponse à l'irradiation, pour au moins un triplet de paramètres d'irradiation (fmod, λ, P) de la configuration d'irradiation (Cirrad) ;
**Caractérisé par** :
iii) calculer au moins un coefficient de corrélation (R(fmod, λ, P)) entre l'amplitude (A(fmod, λ, P)) et la phase (ϕ(fmod, λ, P)) pour au moins un triplet de paramètres d'irradiation (fmod, λ, P) de la configuration d'irradiation (Cirrad) sur une période de fenêtre (Twin) comprenant au moins deux instants d'irradiation avec l'émetteur lumineux ;
iv) comparer au moins un coefficient de corrélation (R(fmod, λ, P)) à au moins une valeur seuil (Rthresh) ;
v) fournir un résultat de test basé sur le résultat de la comparaison.

9. Programme informatique comprenant des instructions permettant au dispositif de surveillance d'analyte (1) de la revendication 8 d'acquérir des données de mesurs obtenues à l'étape c) de la revendication 1 et d'exécuter les étapes d) à f) du processus de test in situ de la revendication 1 ou le procédé de test in situ de l'une quelconque des revendications 2 à 6.
